# EUROPEAN PATENT APPLICATION

(11) **EP 3 939 519 A1**
(43) Date of publication of application: **19.01.2022**
(21) Application number: 21185236.3
(22) Date of filing: 13.07.2021
(51) Int. Cl.: A61B 17/072, A61B 90/30

(54) **SURGICAL STAPLER WITH ILLUMINATION**

(30) Priority: 14.07.2020 US 202063051433 P; 04.05.2021 US 202117307168
(71) Applicant: Covidien LP, Mansfield, MA 02048 (US)
(72) Inventor: GEORGE, Sabastian K., 562125 Bangalore (IN); VADALI, K V S Manoj Kumar, 502032 Hyderabad (IN)
(74) Representative: Maschio, Antonio

(57) **Abstract**

A surgical stapler includes a handle assembly and an end effector connected by an elongate body. The end effector includes a cartridge assembly having staples, an anvil to assist forming staples, and illuminating devices on the end effector capable of illuminating tissue. The illuminating devices on the end effector assist in both illuminating the tissue to be stapled and to visualizing blood vessels in the tissue, including veins.

## Description

### CROSS REFERENCE TO RELATED APPLICATION

This application claims the benefit of the filing date of provisional U.S. Application No. 63/051,433, filed on July 14, 2020.

### TECHNICAL FIELD

The disclosure relates to a surgical stapler for implanting mechanical surgical fasteners into the tissue of a patient and, in particular, to a surgical stapler which illuminates tissue being stapled to identify the presence of blood vessels, including veins and arteries.

### BACKGROUND

Minimally invasive surgery, such as endoscopic surgery, reduces the invasiveness of surgical procedures. Endoscopic surgery involves surgery through body walls, for example, viewing and/or operating on the ovaries, uterus, gall bladder, bowels, kidneys, appendix, etc. There are many common endoscopic surgical procedures, including arthroscopy, laparoscopy, gastroentroscopy and laryngobronchoscopy, just to name a few. In these procedures, trocars are utilized to pass through incisions and penetrate the abdominal wall, thereby permitting endoscopic surgery to be performed. Trocar tubes or cannula devices are extended into and left in place in the abdominal wall to provide access for endoscopic surgical tools. A camera or endoscope is inserted through a trocar tube to permit the visual inspection and magnification of a body cavity. The surgeon can then perform diagnostic and/or therapeutic procedures at the surgical site with the aid of specialized instrumentation, such as forceps, graspers, cutters, applicators, and the like, which are designed to fit through additional cannulas.

One issue which may arise with minimally invasive procedures is vessel identification and dissection. This issue may be more prevalent with inexperienced laparoscopic surgeons. Moreover, it is time consuming in dissection to approach certain locations and body conditions. Differences in a patient's anatomy can contribute to the need to convert a laparoscopic procedure to an open procedure.

Improved devices to assist in visualization of the surgical field remain desirable.

### SUMMARY

A surgical stapler of the disclosure includes a handle assembly, an end effector coupled to the handle assembly, and illuminating devices on the end effector capable of illuminating tissue. The end effector includes a first jaw member including a cartridge assembly and a second jaw member including an anvil portion. The first and second jaw members are moveable relative to one another between an open position and a clamped position. The illuminating devices on the end effector assist in both illuminating the tissue to be stapled and to visualizing blood vessels in the tissue, including veins and/or arteries.

In aspects, a surgical stapler includes a handle assembly, an end effector including a cartridge assembly, an anvil, and an illuminating device. The surgical stapler also has an elongate body connecting the handle assembly and the end effector. Application of light from the illuminating device reveals blood vessels in tissue.

In other aspects, a surgical stapler of the disclosure includes a handle assembly, an end effector including a cartridge assembly, an anvil, and an illuminating device on the cartridge assembly. The surgical stapler also has an elongate body connecting the handle assembly and the end effector. Application of light from the illuminating device reveals blood vessels in tissue.

In yet other aspects, a surgical stapler includes a handle assembly, an end effector including a cartridge assembly, and an anvil. The surgical stapler also has an elongate body connecting the handle assembly and the end effector, and a first sleeve encompassing the cartridge assembly, the first sleeve including an illuminating device. Application of light from the illuminating device reveals blood vessels in tissue. In some aspects, the surgical stapler includes a second sleeve encompassing the anvil, the second sleeve including an illuminating device.

### BRIEF DESCRIPTION OF THE DRAWINGS

Aspects of the disclosure are described herein with reference to the accompanying drawings, wherein:
FIG. 1 is a side perspective view illustrating a surgical stapler including a handle assembly, a shaft portion, and an end effector;
FIG. 2 is a perspective view, with parts separated, illustrating the end effector of the surgical stapler of FIG. 1;
FIG. 3 illustrates the surgical stapler of FIG. 1 in use;
FIG. 4 illustrates the surgical stapler of FIG. 1 in an illuminated state;
FIG. 5 illustrates an alternate aspect of the surgical stapler of FIG. 1, depicting sleeves that may be placed around a cartridge assembly and an anvil of the surgical stapler, the sleeves capable of illuminating tissue; and
FIG. 6 is a side view illustrating the end effector of the surgical stapler of FIG. 5 in an illuminated state.

### DETAILED DESCRIPTION

The disclosed surgical staplers are described in detail with reference to the drawings, in which like reference numerals designate identical or corresponding elements in each of the several views. As used herein the term "distal" refers to that portion of the surgical stapler, or component thereof, farther from the user, while the term "proximal" refers to that portion of the surgical stapler, or component thereof, closer to the user.

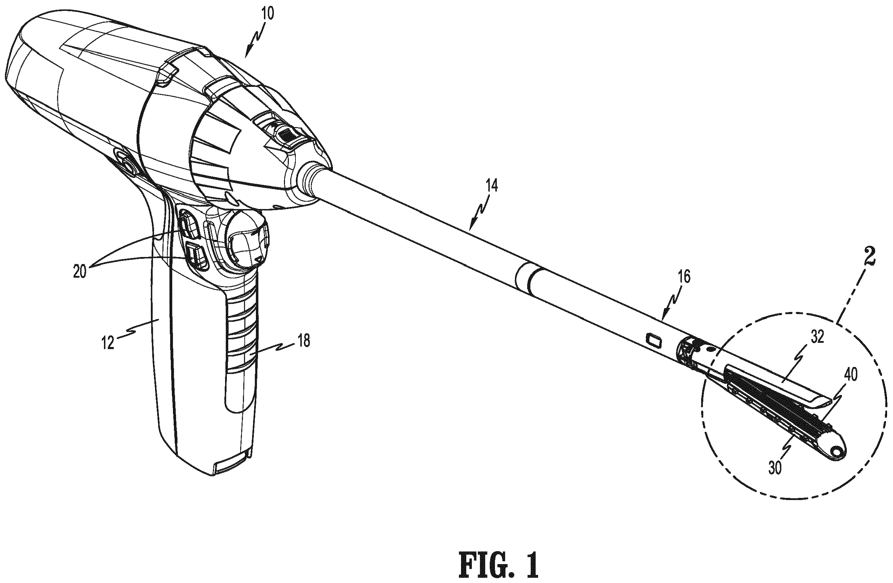

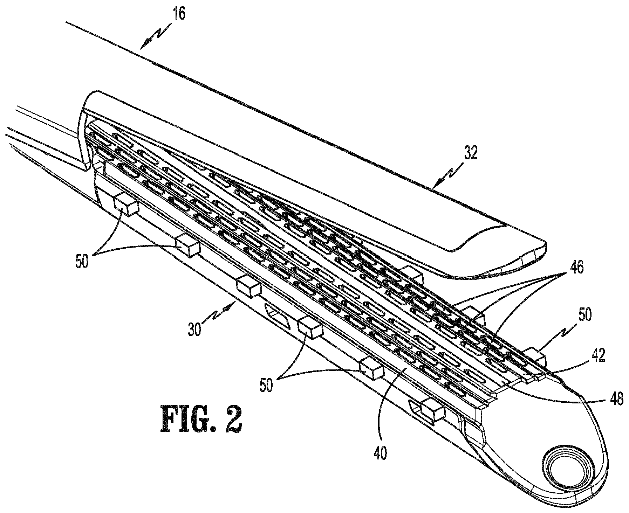

With reference to FIGS. 1 and 2, a surgical stapler in accordance with an aspect of the disclosure is shown generally as surgical stapler 10 that includes a handle assembly 12, an elongate body or adapter 14, and an end effector 16. In the illustrated aspect, the handle assembly 12 is powered and includes a stationary handgrip 18 and actuation buttons 20. The actuation buttons 20 are operable to actuate various functions of the end effector 16 via the adapter 14, including approximation, stapling, and dissection. In aspects of the disclosure, the handle assembly 12 supports batteries (not shown) that provide energy to the handle assembly 12 to operate the surgical stapler 10. Although the surgical stapler 10 is illustrated as a powered surgical stapler, it is envisioned that the advantages of this disclosure are suitable for use with manually powered surgical staplers as well as robotically controlled surgical staplers.

According to the disclosure, the end effector 16 includes a cartridge assembly 30 having one or more surgical fasteners and a second opposing jaw member including an anvil 32 for deploying and forming the staples. As is known in the art, the cartridge assembly 30 and the anvil 32 are coupled together such that the end effector 16 can pivot between an open position and a clamped position. The cartridge assembly 30 includes an exemplary aspect of the disclosed staple cartridge shown generally as staple cartridge 40.

In certain aspects of the disclosure, the staples are housed in cartridge assembly 30 to apply linear rows of staples to body tissue either in simultaneous or sequential manner. Either one or both of the anvil 32 and the cartridge assembly 30 are movable in relation to one another between an open position in which the anvil 32 is spaced from cartridge assembly 30 and an approximated or clamped position in which the anvil 32 is in juxtaposed alignment with cartridge assembly 30.

FIG. 2 illustrates the end effector 16 and the staple cartridge 40 which includes a cartridge body 42 and a plurality of staples (not shown). The cartridge body 42 defines a plurality of staple pockets 46 and a central knife slot 48 that extends along a midline of the cartridge body 42. As known in the art, the central knife slot 48 facilitates translation of a knife bar (not shown) through the end effector 16 to eject staples supported within a staple cartridge and cut tissue clamped between the cartridge assembly 30 and anvil 32. U.S. Patent No. 5,865,361 discloses a manually powered surgical stapler including a knife bar that is movable through the tool assembly to eject staples from a staple cartridge and to cut tissue clamped between an anvil and a cartridge assembly of the surgical stapler. Although the cartridge body 42 is illustrated as being linear, it is envisioned that the cartridge body 42 may have a non-linear configuration or curved along its longitudinal axis.

It is envisioned that the staple pockets 46 in the cartridge body 42 need not be arranged in rows as illustrated above but rather may be arranged in a variety of different patterns in the cartridge body.

In aspects of the disclosure, an illuminating device is included on the end effector to identify the presence of veins and/or arteries at the time of dissection inside the body. The illuminating device is illuminated below the tissue that is intended to be cut, which, when viewed from above the tissue, allows for visualization of major veins and/or arteries.

For example, as shown in FIG. 2, the cartridge assembly 30 possesses illuminating devices 50 which may be illuminated in use. Illumination of the illuminating devices 50 on the cartridge assembly 30 permits visualization of major veins and/or arteries within the tissue, so that the surgeon operating the surgical stapler may adjust the placement of staples within the patient's tissue, thereby minimizing bleeding from unnecessarily severing veins and/or arteries within the tissue.

The illuminating devices 50 may emit light of any suitable wavelength that assists in the visualization of veins and/or arteries within the tissue being dissected. In aspects, the illuminating devices 50 are light emitting diodes ("LED") of a bright red color which are integrated on the cartridge assembly 30 of the end effector 16 of the surgical stapler 10. Other colors may be used, including blue, green, etc. In some aspects, the illuminating devices 50 are light emitting diodes capable of changing color (not shown), from red, to blue, to green, etc.

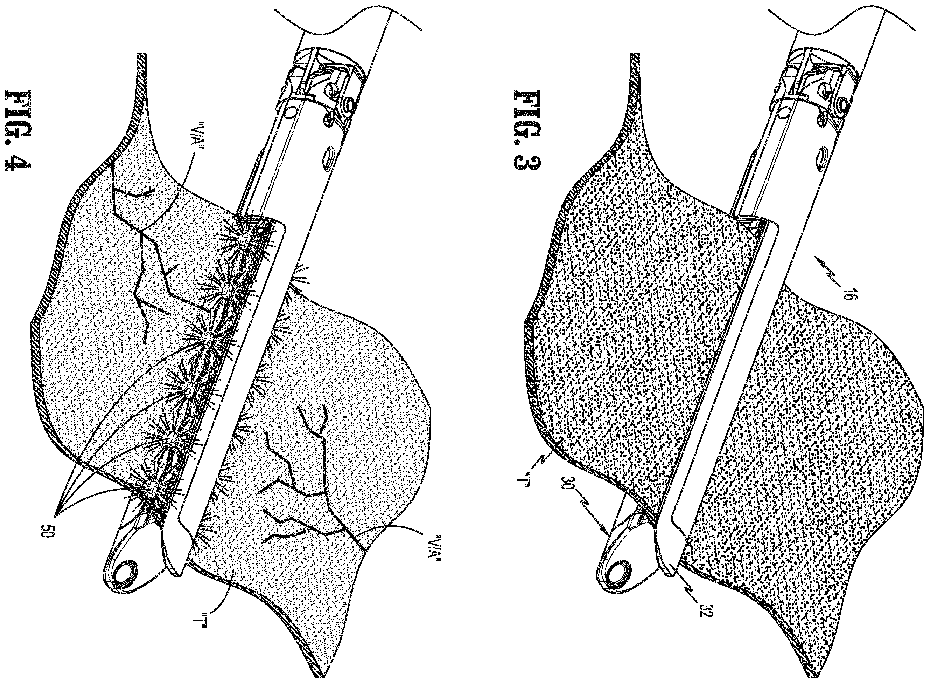

With reference to FIGS. 3 and 4, operation of the surgical stapler 10 will now be described. The trigger (not shown) is depressed so that tissue "T" is encompassed between the anvil 32 and the cartridge assembly 30 of the end effector 16 and the anvil 32 and cartridge assembly 30 close about the tissue "T". At this point, as shown in FIG. 4, the surgeon activates the illuminating devices 50, so that major veins/arteries "V/A" are visible.

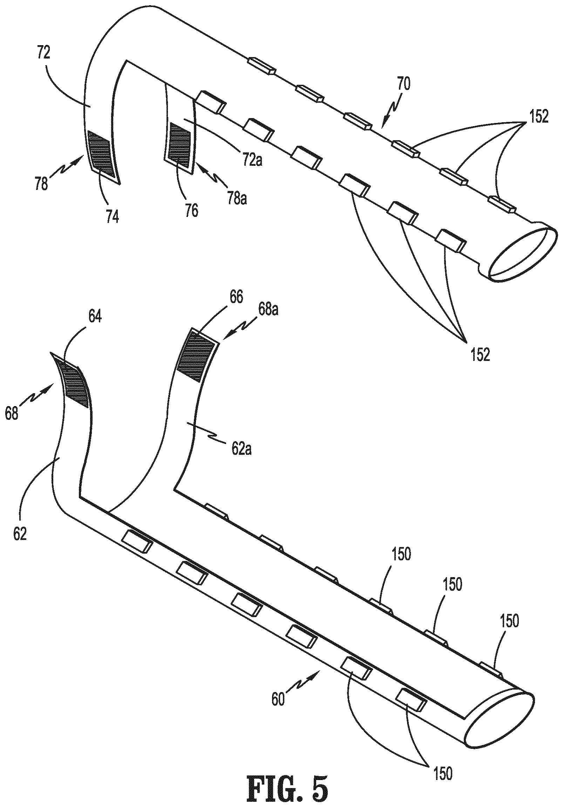

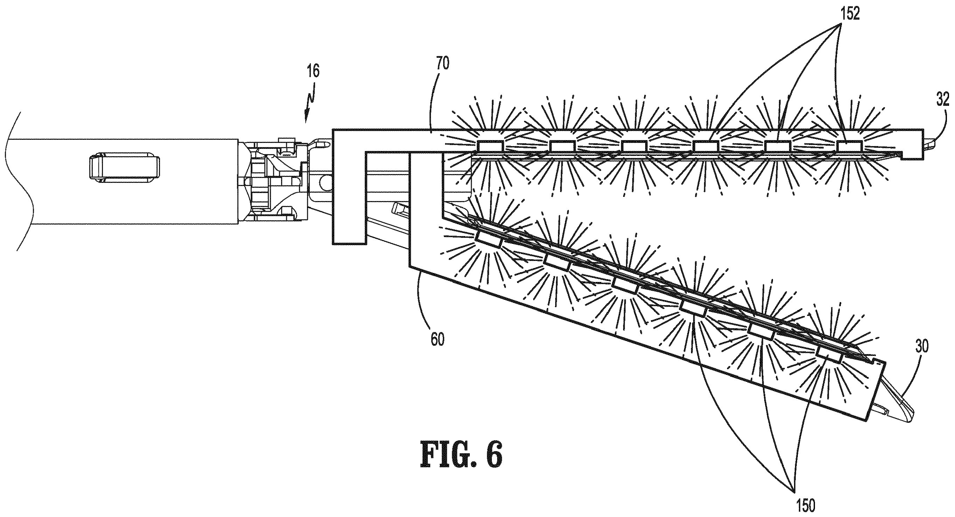

In alternate aspects, as depicted in FIGS. 5 and 6, a first sleeve 60 may be constructed for placement over the cartridge assembly 30 and a second sleeve 70 may be constructed for placement over the anvil 32. The first sleeve 60 has illuminating devices 150 on an outer surface thereof, and the second sleeve 70 has illuminating devices 152 on an outer surface thereof. In this way, existing surgical staplers may be retrofitted with the first sleeve 60 and the second sleeve 70, so that a surgeon may activate the illuminating devices as described above to illuminate tissue, as well as veins and/or arteries within the tissue. Placement of the first sleeve 60 over the cartridge assembly 30 and the second sleeve 70 over the anvil 32, minimizes the need to orient the illuminating devices 150, 152 within the patient to illuminate tissue and permits enhanced viewing of the illuminated tissue, including veins and/or arteries within the tissue.

The first sleeve 60 and the second sleeve 70 can be independently controlled (not shown), so that a surgeon may activate the illuminating devices 150 on the first sleeve 60, or the illuminating devices 152 on the second sleeve 70, but not necessarily both. In use, the surgeon would activate whichever illuminating devices are under the tissue, as described above, in order to illuminate the tissue.

While FIGS. 5 and 6 depict the first sleeve 60 for placement over the cartridge assembly 30 and the second sleeve 70 for placement over the anvil 32, it is to be understood that, in some aspects (not shown), the first sleeve 60 may be present for placement over the cartridge assembly 30, without the need for the second sleeve 70 for placement over the anvil 32.

As depicted in FIG. 5, the first sleeve 60 includes tabs 62, 62a for attaching the first sleeve 60 to the cartridge assembly 30 and the second sleeve 70 includes tabs 72, 72a for attaching the second sleeve 70 to the anvil 32. As depicted in FIG. 5, in aspects tabs 62, 62a on the first sleeve 60 include hook and loop fasteners 64, 66 (including, for example, those commercially sold as VELCRO fasteners) at free ends 68, 68a of the tabs 62, 62a on the first sleeve 60 to attach the first sleeve 60 to the cartridge assemble 30. Similarly, tabs 72, 72a on the second sleeve 70 include hook and loop fasteners 74, 76 (including, for example, those commercially sold as VELCRO fasteners) at free ends 78, 78a of the tabs 72, 72a on the second sleeve 70 to attach the second sleeve 70 to the anvil 32.

The first sleeve 60 and the second sleeve 70 may have their own power source (not shown) for illuminating the illuminating devices 150 and 152, respectively. In the alternative, the first sleeve 60 and the second sleeve 70 include connectors (not shown) for connecting the first sleeve 60 and the second sleeve 70 to the stapler 10, by which power is supplied to the first sleeve 60 and the second sleeve 70 and allows the surgeon to illuminate the illuminating devices 150 and 152, respectively.

Any of the components described herein may be fabricated from either metals, plastics, resins, composites or the like taking into consideration strength, durability, wearability, weight, resistance to corrosion, ease of manufacturing, cost of manufacturing, and the like.

The disclosed devices help reduce the complexity of the surgical procedure, as they prevent accidental cutting of veins and/or arteries. The reduction in complexity, including the avoidance of accidental cutting of veins and/or arteries, will reduce the duration of the surgical procedure and enhance recovery time for the patient.

It will be understood that various modifications may be made to the disclosed surgical staplers. Therefore, the above description should not be construed as limiting, but merely as exemplifications of aspects of the disclosure. Those skilled in the art will envision other modifications within the scope and spirit of the disclosure. For example, any and all features of one described aspect may be suitably incorporated into another aspect.

The invention may be described by reference to the following numbered paragraphs:-
1. A surgical stapler, comprising:
   a handle assembly;
   an end effector including a cartridge assembly, an anvil, and an illuminating device; and
   an elongate body connecting the handle assembly and the end effector,
   wherein application of light from the illuminating device reveals blood vessels in tissue.
2. The surgical stapler according to paragraph 1, wherein the illuminating device includes a plurality of illuminating devices.
3. The surgical stapler according to paragraph 1, wherein the illuminating device includes light emitting diodes.
4. The surgical stapler according to paragraph 3, wherein the light emitting diodes are red.
5. The surgical stapler according to paragraph 1, wherein the cartridge assembly includes the illuminating device.
6. The surgical stapler according to paragraph 1, further comprising a first sleeve encompassing the cartridge assembly, the first sleeve including the illuminating device.
7. The surgical stapler according to paragraph 6, further comprising a second sleeve encompassing the anvil, the second sleeve including the illuminating device.
8. A surgical stapler, comprising:
   a handle assembly;
   an end effector including a cartridge assembly, an anvil, and an illuminating device on the cartridge assembly; and
   an elongate body connecting the handle assembly and the end effector,
   wherein application of light from the illuminating device reveals blood vessels in tissue.
9. The surgical stapler according to paragraph 8, wherein the illuminating device includes a plurality of illuminating devices.
10. The surgical stapler according to paragraph 8, wherein the illuminating device includes light emitting diodes.
11. The surgical stapler according to paragraph 10, wherein the light emitting diodes are red.
12. A surgical stapler, comprising:
   a handle assembly;
   an end effector including a cartridge assembly, and an anvil;
   an elongate body connecting the handle assembly and the end effector; and
   a first sleeve encompassing the cartridge assembly, the first sleeve including an illuminating device,
   wherein application of light from the illuminating device reveals blood vessels in tissue.
13. The surgical stapler according to paragraph 12, wherein the illuminating device includes a plurality of illuminating devices.
14. The surgical stapler according to paragraph 12, wherein the illuminating device includes light emitting diodes.
15. The surgical stapler according to paragraph 14, wherein the light emitting diodes are red.
16. The surgical stapler according to paragraph 12, further comprising a second sleeve encompassing the anvil, the second sleeve including an illuminating device.

## Claims

1. A surgical stapler, comprising:
a handle assembly;
an end effector including a cartridge assembly, an anvil, and an illuminating device; and
an elongate body connecting the handle assembly and the end effector,
wherein application of light from the illuminating device reveals blood vessels in tissue.

2. The surgical stapler according to claim 1, wherein the illuminating device includes a plurality of illuminating devices.

3. The surgical stapler according to claim 1, wherein the illuminating device includes light emitting diodes.

4. The surgical stapler according to claim 3, wherein the light emitting diodes are red.

5. The surgical stapler according to any preceding claim, wherein the cartridge assembly includes the illuminating device.

6. The surgical stapler according to any preceding claim, further comprising a first sleeve encompassing the cartridge assembly, the first sleeve including the illuminating device.

7. The surgical stapler according to claim 6, further comprising a second sleeve encompassing the anvil, the second sleeve including the illuminating device.

8. A surgical stapler, comprising:
a handle assembly;
an end effector including a cartridge assembly, an anvil, and an illuminating device on the cartridge assembly; and
an elongate body connecting the handle assembly and the end effector,
wherein application of light from the illuminating device reveals blood vessels in tissue.

9. The surgical stapler according to claim 8, wherein the illuminating device includes a plurality of illuminating devices.

10. The surgical stapler according to claim 8 or claim 9, wherein the illuminating device includes light emitting diodes.

11. The surgical stapler according to claim 10, wherein the light emitting diodes are red.

12. A surgical stapler, comprising:
a handle assembly;
an end effector including a cartridge assembly, and an anvil;
an elongate body connecting the handle assembly and the end effector; and
a first sleeve encompassing the cartridge assembly, the first sleeve including an illuminating device,
wherein application of light from the illuminating device reveals blood vessels in tissue.

13. The surgical stapler according to claim 12, wherein the illuminating device includes a plurality of illuminating devices.

14. The surgical stapler according to claim 12 or claim 13, wherein the illuminating device includes light emitting diodes; preferably wherein the light emitting diodes are red.

15. The surgical stapler according to any of claims 8 to 14, further comprising a second sleeve encompassing the anvil, the second sleeve including an illuminating device.
